# EUROPÄISCHE PATENTSCHRIFT

(11) **EP 2 442 098 B1**
(45) Veröffentlichungstag und Bekanntmachung des Hinweises auf die Patenterteilung: **23.05.2018**
(21) Anmeldenummer: 11005541.5
(22) Anmeldetag: 07.07.2011
(51) Int. Cl.: G01N 25/20, G01K 17/08, G01N 15/08, G01N 33/28, G01N 15/06

(54) **VERFAHREN UND VORRICHTUNG ZUR BESTIMMUNG DER RUSSKONZENTRATION IM MOTORÖL VON BRENNKRAFTMASCHINEN**
METHOD AND DEVICE FOR DETERMINING THE CONCENTRATION OF CARBON BLACK IN MOTOR OIL OF INTERNAL COMBUSTION ENGINES
PROCÉDÉ ET DISPOSITIF DE DÉTERMINATION DE LA CONCENTRATION EN SUIE DANS L'HUILE DE MOTEURS À COMBUSTION INTERNE

(30) Priorität: 16.10.2010 DE 102010048748
(43) Veröffentlichungstag der Anmeldung: 18.04.2012
(73) Patentinhaber: MAN Truck & Bus AG, 80995 München (DE)
(72) Erfinder: Beck, Harald, Dr., 90459 Nürnberg (DE); Fischer, Felix, 90409 Nürnberg (DE)
(74) Vertreter: Liebl, Thomas

(56) Entgegenhaltungen:
- US-A- 4 281 533
- US-A- 5 309 213
- US-A- 5 332 961
- US-A- 5 438 420

## Beschreibung

Die Erfindung betrifft ein Verfahren zur Bestimmung der Russkonzentration im Motoröl von Brennkraftmaschinen nach dem Oberbegriff des Anspruchs 1. Ferner betrifft die Erfindung eine Vorrichtung zur Bestimmung der Russkonzentration im Motoröl von Brennkraftmaschinen nach dem Oberbegriff des Anspruchs 6.

Es ist allgemein bekannt, dass sich der Eintrag von Russ in das Motoröl von Brennkraftmaschinen negativ auf die Öleigenschaften auswirken kann. Beispielsweise kann ein vermehrter Russeintrag in das Motoröl eine Verschlechterung der Viskosität bzw. der Schmierfähigkeit des Motoröls bewirken. Mit der auf Grund der modernen Abgasgesetzgebung zunehmend komplexer werdenden Abgasnachbehandlungserfordernisse werden moderne Brennkraftmaschinen jedoch vermehrt in solchen Betriebspunkten betrieben, bei denen ein erhöhter Russeintrag ins Motoröl unvermeidbar ist. Ein derartiger Eintrag von Russ in das Motorenöl kann daher die Ölwechselintervalle verkürzen, was jedoch den Kundenwünschen nach immer längeren Ölwechselintervallen zuwiderläuft.

Es besteht daher ein Bedürfnis, den Russeintrag in das Motoröl von Brennkraftmaschinen, insbesondere von Diesel-Brennkraftmaschinen in Nutzfahrzeugen, auf funktionssichere Weise relativ genau vorhersagen zu können, um für bestimmte Betriebsbedingungen den Russeintrag über die Laufzeit der Brennkraftmaschine hochrechnen zu können.

Aus der US 5,309,213 A ist bereits ein Verfahren und eine Vorrichtung bekannt, mittels der die Konzentration der optisch dämpfenden Materialien in Flüssigkeiten, wie zum Beispiel Russ in Schmieröl, bestimmt werden können. Konkret wird hierzu vorgeschlagen, in Verbindung mit einer dünnen, optischen Zelle im Motoröl Licht einzukoppeln und dadurch die optische Dämpfung an unterschiedlichen Stellen zu messen.

Das US-Patent US 5,322,961 beschreibt einen On-Board-Sensor und ein System zur Ermittlung der Qualität des in einem Verbrennungsmotor als Schmiermittel verwendeten Öles.

US 5,309,213 lehrt ein Verfahren zur optischen Bestimmung der Russmenge in einer Ölprobe. Aus der US 5,438,420 ist eine Vorrichtung zur Überwachung eines Flüssigkeitsverunreinigunggrades beschrieben wobei Lichtenergie auf eine Flüssigkeitsdurchgangseinrichtung fokussiert wird.

Das US-Patent US 4,281,533 lehrt ein Verfahren das die Verwendung einer Energiequelle zur Beaufschlagung des überprüften Motoröls und einer Temperaturmesseinrichtung zur Messung der absorbierten Energiemenge vorsieht.

Demgegenüber ist es Aufgabe der vorliegenden Erfindung, ein Verfahren und eine Vorrichtung zur Bestimmung der Russkonzentration im Motoröl von Brennkraftmaschinen zu schaffen, mittels der eine Russkonzentration bzw. nachfolgend ein Russeintrag über eine bestimmte Brennkraftmaschinenlaufzeit auf einfache Weise sowie funktionssicher ermittelt werden kann.

Diese Aufgabe wird gelöst mit den Merkmalen der unabhängigen Patentansprüche. Vorteilhafte Ausgestaltungen hierzu sind Gegenstand der darauf rückbezogenen Unteransprüche.

Mit dieser erfindungsgemäßen Verfahrensführung wird somit eine Russkonzentration auf der Basis derjenigen Energiemenge bestimmt, die im Motoröl absorbiert wird, was eine besonders zuverlässige und funktionssichere Bestimmung der Russkonzentration und damit nachfolgend des Russeintrags in ein Motoröl erlaubt.

Gemäß einer bevorzugten Verfahrensführung kann vorgesehen sein, dass zur Bestimmung der Russkonzentration als weiterer Parameter die Fließgeschwindigkeit des Motoröls im Bereich der Messstrecke und/oder die spezifische Wärmekapazität des Motoröls und/oder der Absorptionsquerschnitt von Russ, beispielsweise in Form des totalen Wirkungsquerschnitts für die Strahlungsabsorption der Russpartikel, herangezogen wird. Dadurch lässt sich die Aussagegenauigkeit bei der Bestimmung der Russkonzentration noch weiter erhöhen.

Insgesamt kann somit mit einer erfindungsgemäßen Verfahrensführung die Russkonzentration im Motoröl auf einfache und funktionssichere Weise zuverlässig bestimmt werden, insbesondere in Abhängigkeit von definierten Betriebspunkten bestimmt werden. Ferner können alternativ oder zusätzlich auch Katalysatorbetriebspunkte und/oder Regenerationsmaßnahmen für Katalysatoren und/oder Partikelfilter auf einfache Weise bestimmt werden.

Besonders bevorzugt handelt es sich bei der Energiequelle um eine optische Energiequelle, wie zum Beispiel einen Laser, zum Beispiel einen Diodenlaser, welche Energiequelle in wenigstens einem definierten messstreckenseitigen Einstrahlpunkt oder -bereich Energie einstrahlt. Gemäß einer bevorzugten Ausgestaltung der vorliegenden Erfindung ist vorgesehen, dass die Messstrecke durch eine Durchflussküvette gebildet ist, durch die eine definierte Menge des Motorenöls geleitet wird, wobei vorgesehen ist, dass die Temperaturmessstellen im Bereich voneinander beabstandeter Küvettenbereiche angeordnet sind. Die Temperaturerfassung erfolgt hier dann bevorzugt dergestalt, dass, zum Beispiel mittels einer Differenztemperaturermittlung, die Temperaturdifferenz stromauf und stromab des messstreckenseitigen Einstrahlpunktes bzw. -bereiches ermittelt und ausgewertet wird. Hierzu strahlt dann die Energiequelle im Bereich zwischen den beiden voneinander beabstandeten Temperaturmessstellen Energie in das Motoröl ein.

Grundsätzlich ist davon auszugehen, dass der das Ölvolumen quer zur Fließrichtung vollständig durchstrahlende Laserstrahl einen, über die gesamte Querschnittsfläche des Ölvolumens im Einstrahlpunkt, gleichmäßigen Energieeintrag bewirkt, wobei diese Energie, bezogen auf die Querschnittsfläche, im wesentlichen gleichmäßig von den Russpartikeln absorbiert wird. Anschließend wird diese Energie unter, bezogen auf die Querschnittsfläche, gleichmäßiger Erhöhung der Motoröltemperatur direkt an das Motoröl abgegeben, so dass der Einsatz von einem Sensor zur Temperaturerfassung an der wenigstens einen Temperaturmessstelle, insbesondere an einer im Bereich der stromab des Einstrahlpunktes bzw. - bereiches liegenden Temperaturmessstelle ausreichend ist. Insbesondere bei höheren zu erwartenden Russkonzentrationen, bei denen der Laserstrahl das Ölvolumen gegebenenfalls nicht mehr vollständig durchstrahlt bzw. durchstrahlen kann, weil die Russpartikel die Energie bereits frühzeitig absorbieren, hat es sich jedoch als vorteilhaft erwiesen, im Bereich der wenigstens einen Temperaturmessstelle mehrere voneinander räumlich beabstandete Temperatursensoren anzuordnen, mittels denen eine gegebenenfalls vorhandene räumlich unterschiedliche Temperaturverteilung im Motoröl, zum Beispiel im Bereich oder stromab des Einstrahlpunktes oder - bereiches, einfach und zuverlässig erfasst werden kann. Hierzu werden bevorzugt die Temperatursensoren in einer, bezogen auf die Fließrichtung des Motoröls, gleichen Querschnittsebene angeordnet und/oder weisen die Temperatursensoren einen in Fließrichtung im Wesentlichen gleichen Abstand zum Einstrahlbereich auf.

Besonders bevorzugt ist eine Verfahrensführung, bei der die Russkonzentration für definierte Betriebszustände der Brennkraftmaschine kontinuierlich oder aber auch intervallartig, zum Beispiel periodisch, über die Betriebsdauer der Brennkraftmaschine erfasst und einer Auswerteeinrichtung zugeführt wird, in der ein laufzeitabhängiger und/oder last- bzw. betriebspunktabhängiger Russeintrag in das Motorenöl bestimmt wird. Mit einer derartig ermittelten Russkonzentration lässt sich auf einfache Weise bestimmen, wann gegebenenfalls mit einem Ölwechsel zu rechnen ist. Gegebenenfalls kann dann eine Betriebsstrategie der Brennkraftmaschine so eingestellt bzw. beeinflusst werden, dass ein Ölwechsel zu einem definierten Zeitpunkt erforderlich wird.

Die Aufgabe wird bezüglich der Vorrichtung ferner mit den Merkmalen des Anspruchs 6 gelöst.

Die sich mit einer derartigen Vorrichtung ergebenden Vorteile wurden bereits zuvor ausführlich erläutert.

Gemäß einer konkreten Ausgestaltung können die Temperaturmessstellen jeweils wenigstens einen durch eine Spule gebildeten Temperatursensor umfassen.

Ein Ausführungsbeispiel zur konkreten Umsetzung einer erfindungsgemäßen Vorrichtung und einer erfindungsgemäßen Verfahrensführung ist in den Figuren schematisch dargestellt.

Es zeigen:
- Fig.1: schematisch eine beispielhafte nicht-erfindungsgemäße Ausführungsform einer Vorrichtung zur Bestimmung des Russeintrags in Motoröl,
- Fig. 2: schematisch einen erfindungsgemäßen Aufbau mit mehreren stromab des Einstrahlpunktes angeordneten Temperatursensoren, und
- Fig. 3: schematisch eine weitere alternative Variante einer nichterfindungsgemäßen Vorrichtung.

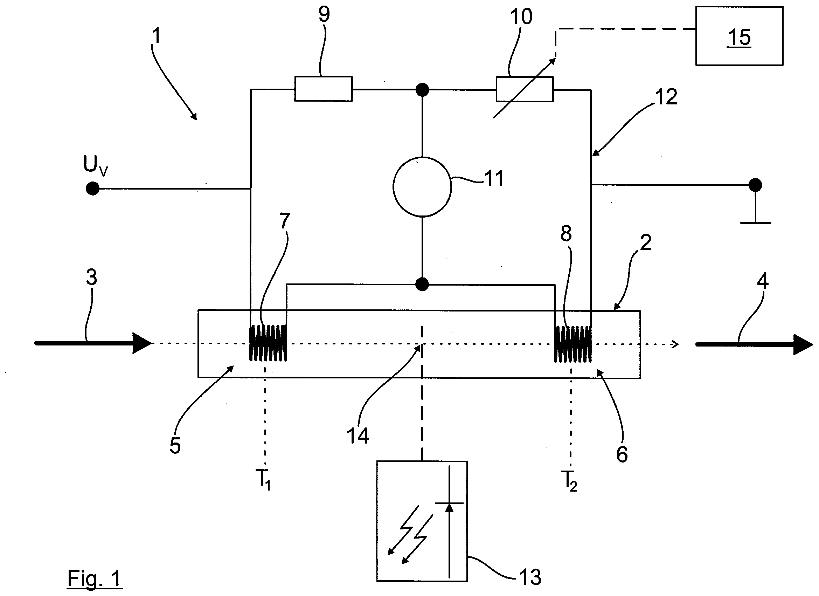

In Fig. 1 ist ein Sensor 1 zur Bestimmung der Russkonzentration im Motoröl einer Brennkraftmaschine gezeigt, welcher Sensor 1 eine Durchflussküvette 2 aufweist, die ein definiertes, vorgegebenes Volumen aufweist. Diese Durchflussküvette 2 wird in Richtung der Strömungspfeile 3, 4 für eine bestimmte Zeitdauer von einer vorgegebenen Menge eines Motoröls einer Brennkraftmaschine durchströmt, wobei die Durchflussgeschwindigkeit des Motoröls durch die Durchflussküvette 2 im Wesentlichen stets gleich ist und einen vorgegebenen und damit bekannten Geschwindigkeitsbetrag aufweist.

Im einströmseitigen Küvettenbereich 5 ist hier beispielhaft eine erste Drahtspule 7 im Strömungsweg des Motoröls angeordnet. Beabstandet dazu ist im ausströmseitigen Küvettenbereich 6 eine weitere im Wesentlichen baugleiche Drahtspule 8 angeordnet, die ebenfalls im Strömungsweg des Motoröls liegt.

Diese beiden Spulen 7, 8 bilden zusammen mit den Widerständen 9, 10, von denen der Widerstand 10 als veränderbarer Widerstand ausgebildet ist, sowie mit einem Spannungsmessgerät 11 eine an sich bekannte Brückenschaltung 12 aus, die hier lediglich beispielhaft und schematisch dargestellt ist und selbstverständlich auch jeden anderen geeigneten Aufbau aufweisen kann.

Mit einer derartigen Brückenschaltung 12 werden im Bereich der Spulen 7, 8 zwei voneinander beabstandete Temperaturmessstellen bzw. -sensoren ausgebildet, mittels denen die Temperaturen des Motoröls im einströmseitigen Küvettenbereich 5 einerseits sowie im ausströmseitigen Küvettenbereich 6 andererseits erfasst werden können. Konkret wird bei der hier gewählten und gezeigten Brückenschaltung 12 somit die Temperaturabhängigkeit des elektrischen Widerstandes von Leitern zur Messung der Temperatur ausgenutzt. Dies ist allgemein bekannt und braucht an dieser Stelle nicht weiter erörtert zu werden.

Der Sensor 1 umfasst des Weiteren als optische Energiequelle einen hier beispielhaft gewählten Diodenlaser 13, mittels dem, wie dies in Fig. 1 lediglich strichliert dargestellt ist, im Bereich zwischen den beiden durch die Drahtspulen 7, 8 gebildeten Temperaturmessstellen optische Energie dergestalt in die Durchflussküvette 2 eingestrahlt wird, dass die im Motoröl enthaltenen Russpartikel diese Energie absorbieren sowie anschließend unter Erhöhung der Motoröltemperatur wieder an das Motoröl abgeben. Dadurch wird im Bereich der Drahtspule 8 eine Temperatur T₂ gemessen, die in einem definierten Maße größer ist als die Temperatur T₁, die im Bereich der Drahtspule 7 erfasst und gemessen wird. Auf der Basis dieser Temperaturdifferenz zwischen den beiden Temperaturen T₂ und T₁ an den drahtspulenseitigen Temperaturmessstellen lässt sich mittels einer hier lediglich äußerst schematisch dargestellten Auswerteeinrichtung 15 in Verbindung mit der bekannten Fließgeschwindigkeit des Motoröls im Küvettenbereich, der spezifischen Wärmekapazität des eingesetzten Motoröls sowie des bekannten Absorptionsquerschnitts von Russ bzw. der Russpartikel die jeweilige Russkonzentration im Motoröl für den aktuellen Betriebszustand der Brennkraftmaschine ermitteln und entsprechend betriebspunktabhängig und/ oder über die Lebensdauer der Brennkraftmaschine ein Gesamt-Russeintrag ermitteln.

Die mittige Positionierung des Einstrahlpunkts 14 mit Bezug auf die Position der die Temperaturmessstellen ausbildenden Drahtspulen 7, 8 ist hier lediglich beispielhaft und beliebig gewählt. Auch andere Anordnungen sind selbstverständlich jederzeit möglich.

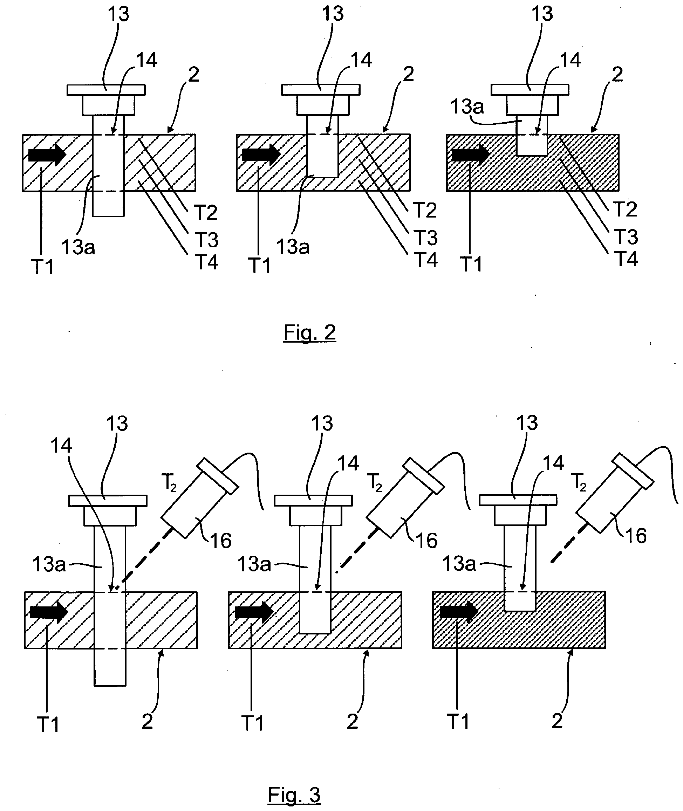

In der Fig. 2 ist schließlich eine erfindungsgemäße Ausführung lediglich äußerst schematisch dargestellt, bei der stromauf des Einstrahlpunkts 14 lediglich ein zum Beispiel durch eine Spule gebildeter Temperatursensor vorgesehen ist, mittels dem die Temperatur T₁ erfasst wird, während stromab des Einstrahlpunkts 14 hier beispielhaft jeweils drei räumlich voneinander beabstandete Temperatursensoren angeordnet sind, mittels denen bezogen auf einen bestimmten in Strömungsrichtung liegenden Küvettenquerschnitt die Temperaturen T₂, T₃ und T₄ an unterschiedlichen Stellen des Querschnitts stromab des Einstrahlpunktes 14 erfasst werden, wodurch es möglich ist, eine über den Strömungsquerschnitt gesehene räumliche Verteilung der Motoröltemperaturen stromab des Einstrahlpunktes 14 festzustellen.

Für den im linken Bild der Fig. 2 dargestellten Fall, dass nur eine normale Rußkonzentration im Motoröl vorliegt, strahlt der Laserstrahl 13a durch den gesamten Messstrecken- bzw. Küvettenbereich durch, so dass im Bereich stromab des Einstrahlpunktes 14 die Temperaturen T₂, T₃ und T₄ im Wesentlichen jeweils den gleichen Temperaturwert aufweisen.

Steigt dagegen die Rußkonzentration im Motoröl so an, dass der Laserstrahl 13a den Messstreckenbereich nicht mehr vollständig durchdringen kann, wie dies im mittleren Bild der Fig. 2 dargestellt ist, so werden im Bereich stromab des Einstrahlpunktes 14 unterschiedliche Temperaturwerte für die Temperaturen T₂ und T₃ einerseits sowie T₄ andererseits ermittelt, das heißt, dass es im hier gezeigten Beispielfall zu keiner oder nur einer unwesentlichen Temperaturerhöhung im Bereich der Position des die Temperatur T₄ erfassenden Temperatursensors gegenüber der stromauf gemessenen Temperatur T₁ kommt.

Das Gleiche ist im rechten Bild der Fig. 2 für einen noch höheren Rußgehalt im Motoröl dargestellt. In diesem Fall wird im Bereich der die Temperaturen T₃ und T₄ erfassenden Temperatursensoren keine bzw. nur eine unwesentliche Abweichung des Temperaturwertes gegenüber der Temperatur T₁ erfasst.

Auf der Basis der räumlichen Ungleichverteilung der Temperatur, bezogen auf einen quer zur Strömungsrichtung liegenden Strömungsquerschnitt kann somit, gegebenenfalls unter Heranziehung der bereits zuvor beschriebenen Parameter oder zumindest wenigstens eines dieser Parameter, auf den Russgehalt im Öl geschlossen werden. Die die Temperatur T₂, T₃ und T₄ erfassenden, hier nicht dargestellten Temperatursensoren liegen dabei bevorzugt in etwa in derselben Querschnittsebene durch die Messstrecke 2.

Der, bezogen auf die Russkonzentration, gleiche Fall ist in der Fig. 3 gezeigt, bei dem jedoch abweichend zur Ausgestaltung nach Fig. 2 nicht stromab des Einstrahlpunktes 14 mehrere zum Beispiel durch Spulen gebildete Temperatursensoren räumlich beabstandet voneinander angeordnet sind, sondern mittels eines Pyrometers 16 die Öltemperatur T₂ direkt am Einstrahlpunkt 14 gemessen wird. Nimmt die Einstrahltiefe des Laserstrahls 13a ab, wie dies in den Bildern der Fig. 3 von links nach rechts gezeigt ist, wird mit der gleichen Energiemenge ein kleineres Ölvolumen erwärmt, welches kleinere Ölvolumen allerdings stärker erwärmt wird und damit auch in der Folge eine höhere Wärmestrahlung abgibt, die dann vom Pyrometer 16 gemessen werden kann. Dadurch kann dann auf den Russgehalt im Öl geschlossen werden.

Es versteht sich, dass die Temperatursensoren in den vorstehend nach den Figuren 2 und 3 beschriebenen Beispielen an den jeweiligen Temperaturmessstellen jeweils auf unterschiedliche Art und Weise ausgebildet sein können, zum Beispiel durch die in Verbindung mit der Fig. 1 gezeigten Spulen, aber auch durch geeignete andere Temperatursensoren wie sie dem Fachmann bekannt sind.

## Patentansprüche

1. Verfahren zur Bestimmung der Russkonzentration im Motoröl von Brennkraftmaschinen, bei dem eine definierte Menge des Motoröls mit einer definierten Fließgeschwindigkeit entlang einer und/oder durch eine Messstrecke (2) geführt wird, wobei das Motoröl im Bereich der Messstrecke (2) dergestalt mit Energie von wenigstens einer Energiequelle (13) in einem definierten Einstrahlpunkt oder -bereich (14) beaufschlagt wird, dass die im Motoröl enthaltenen Russpartikel diese Energie wenigstens zum Teil absorbieren, und dass diese im Messstreckenbereich absorbierte Energiemenge mittels wenigstens einer Messeinrichtung erfasst wird, insbesondere mittels wenigstens einer Temperaturmesseinrichtung erfasst wird und daraus die Russkonzentration im Motoröl bestimmt wird, **dadurch gekennzeichnet, dass** stromauf des Einstrahlpunktes oder-bereiches (14) eine Temperatur (T1) erfasst wird;
dass stromab des Einstrahlpunktes oder-bereiches (14) mehrere Temperaturen (T2, T3, T4) mittels mehreren voneinander räumlich beabstandeten Temperatursensoren erfasst werden, die in einer, bezogen auf die Fließrichtung des Motoröls, gleichen Querschnittsebene angeordnet sind, und
dass auf der Basis der räumlichen Verteilung der erfassten mehreren Temperaturen (T2, T3, T4) auf den Russgehalt im Öl geschlossen wird.

2. Verfahren nach Anspruch 1, **dadurch gekennzeichnet, dass** zur Bestimmung der Russkonzentration als weiterer Parameter die Fließgeschwindigkeit des Motoröls im Bereich der Messstrecke (2) und/oder die spezifische Wärmekapazität des Motoröls und/oder der Absorptionsquerschnitt von Russ herangezogen wird.

3. Verfahren nach einem der vorhergehenden Ansprüche, **dadurch gekennzeichnet, dass** die Energiequelle (13) eine optische Energiequelle ist, insbesondere ein Laser, die in wenigstens einem definierten messstreckenseitigen Einstrahlpunkt oder -bereich (14) Energie einstrahlt.

4. Verfahren nach einem der vorhergehenden Ansprüche, **dadurch gekennzeichnet, dass** die Messstrecke durch eine Durchflussküvette (2) gebildet ist.

5. Verfahren nach einem der vorhergehenden Ansprüche, **dadurch gekennzeichnet, dass** die Russkonzentration für definierte Betriebszustände der Brennkraftmaschine kontinuierlich oder intervallartig über die Betriebsdauer erfasst und einer Auswerteeinrichtung (15) zugeführt wird, in der ein laufzeitabhängiger und/oder lastabhängiger Russeintrag in das Motorenöl bestimmt wird.

6. Vorrichtung zur Bestimmung der Russkonzentration im Motoröl von Brennkraftmaschinen, insbesondere zur Durchführung eines Verfahrens nach einem der vorhergehenden Verfahrensansprüche, mit:
- einer Messstrecke zur Führung einer definierten Menge des Motoröls mit einer definierten Fließgeschwindigkeit;
- wenigstens einer Energiequelle (13), mittels der den Russpartikeln im Motoröl in wenigstens einem definierten Einstrahlpunkt oder -bereich (14) der Messtrecke Energie zuführbar ist;
- eine Temperaturmesseinrichtung zum Erfassen einer Temperatur (T1) stromauf des Einstrahlpunktes oder-bereiches (14);
- einer Temperaturmesseinrichtung zum Erfassen mehrerer Temperaturen (T2, T3, T4) stromab des Einstrahlpunktes oder -bereiches (14) mittels mehrerer voneinander räumlich beabstandeter Temperatursensoren, die in einer, bezogen auf die Fließrichtung des Motoröls, gleichen Querschnittsebene angeordnet sind; und
- einer Auswerteeinrichtung (15) zur Bestimmung der Russkonzentration auf der Basis der räumlichen Verteilung der erfassten mehreren Temperaturen (T2, T3, T4).

7. Vorrichtung nach Anspruch 6, **dadurch gekennzeichnet, dass** die Messstrecke durch eine Durchflussküvette (2) gebildet ist.

8. Vorrichtung nach Anspruch 6, **dadurch gekennzeichnet, dass** die Energiequelle (13) eine optische Energiequelle, insbesondere ein Diodenlaser, ist.

## Claims

1. Method for determining the soot concentration in the engine oil of internal combustion engines, in which a defined quantity of the engine oil is conducted with a defined flow rate along and/or through a measuring section (2), wherein energy is applied to the engine oil in the region of the measuring section (2) by at least one energy source (13) at a defined irradiation point or irradiation region (14), in such a way that the soot particles which are contained in the engine oil at least partially absorb this energy, and that this quantity of energy which is absorbed in the measuring section region is measured by at least one measuring device, in particular by means of at least one temperature-measuring device, and in that the soot concentration in the engine oil is determined therefrom,
**characterized**
**in that** a temperature (T1) is measured upstream of the irradiation point or irradiation region (14); in that a plurality of temperatures (T2, T3, T4) are measured downstream of the irradiation point or irradiation region (14) by means of a plurality of temperature sensors which are spaced apart spatially from one another and are arranged in the same cross-sectional plane with respect to the flowing direction of the engine oil, and
**in that** the soot content in the oil is deduced on the basis of the spatial distribution of the plurality of measured temperatures (T2, T3, T4).

2. Method according to Claim 1, **characterized in that** the flow rate of the engine oil in the region of the measuring section (2) and/or the specific thermal capacity of the engine oil and/or the adsorption cross section of soot are/is used as further parameters for determining the soot concentration.

3. Method according to one of the preceding claims, **characterized in that** the energy source (13) is an optical energy source, in particular a laser, which irradiates energy in at least one defined measuring-section-side irradiation point or irradiation region (14).

4. Method according to one of the preceding claims, **characterized in that** the measuring section is formed by a through-flow cuvette (2).

5. Method according to one of the preceding claims, **characterized in that** the soot concentration is measured continuously or at intervals over the operating period for defined operating states of the internal combustion engine and is fed to an evaluation device (15) in which a running-time-dependent and/or load-dependent soot input into the engine oil is determined.

6. Device for determining the soot concentration in the engine oil of internal combustion engines, in particular for carrying out a method according to one of the preceding method claims, having:
- a measuring section for conducting a defined quantity of the engine oil with a defined flow rate;
- at least one energy source (13) by means of which energy can be fed to the soot particles in the engine oil at at least one defined irradiation point or irradiation region (14) of the measuring section;
- a temperature measuring device for measuring a temperature (T1) upstream of the irradiation point or irradiation region (14);
- a temperature measuring device for measuring a plurality of temperatures (T2, T3, T4) downstream of the irradiation point or irradiation region (14) by means of a plurality of temperature sensors which are spaced apart spatially from one another and are arranged in the same cross-sectional plane with respect to the flowing direction of the engine oil; and
- an evaluation device (15) for determining the soot concentration on the basis of the spatial distribution of the plurality of measured temperatures (T2, T3, T4).

7. Device according to Claim 6, **characterized in that** the measuring section is formed by a through-flow cuvette (2).

8. Device according to Claim 6, **characterized in that** the energy source (13) is an optical energy source, in particular a diode laser.

## Revendications

1. Procédé de détermination de la concentration de suie dans l'huile moteur de moteurs à combustion interne, avec lequel une quantité définie de l'huile moteur est conduite à une vitesse d'écoulement définie le long d'un segment de mesure (2) et/ou à travers celui-ci, l'huile moteur étant chargée dans la zone du segment de mesure (2) avec de l'énergie en provenance d'au moins une source d'énergie (13) en un point ou dans une zone d'irradiation défini(e) de telle sorte que les particules de suie contenues dans l'huile moteur absorbent au moins partiellement cette énergie et que cette quantité d'énergie absorbée dans la zone de du segment de mesure est acquise par le biais d'au moins un appareil de mesure, notamment acquise par le biais d'au moins un appareil de mesure de température, et la concentration de suie dans l'huile moteur étant déterminée à partir de celle-ci,
**caractérisé en ce que**
une température (T1) est acquise en amont du point ou de la zone d'irradiation (14) ;
plusieurs températures (T2, T3, T4) sont acquises en aval du point ou de la zone d'irradiation (14) au moyen de plusieurs capteurs de température espacés les uns des autres dans l'espace, lesquels sont disposés dans un même plan de section transversal en référence au sens d'écoulement de l'huile moteur, et
la teneur en suie dans l'huile est déduite en s'appuyant sur la distribution spatiale des plusieurs températures (T2, T3, T4) acquises.

2. Procédé selon la revendication 1, **caractérisé en ce que** la vitesse d'écoulement de l'huile moteur dans la zone du segment de mesure (2) et/ou la capacité calorifique spécifique de l'huile moteur et/ou la section transversale d'absorption de suie sont utilisées en tant que paramètres supplémentaires pour la détermination de la concentration de suie.

3. Procédé selon l'une des revendications précédentes, **caractérisé en ce que** la source d'énergie (13) est une source d'énergie optique, notamment un laser, qui irradie de l'énergie dans au moins un point ou une zone d'irradiation (14) du côté du segment de mesure.

4. Procédé selon l'une des revendications précédentes, **caractérisé en ce que** le segment de mesure est formé par une cuvette de circulation (2).

5. Procédé selon l'une des revendications précédentes, **caractérisé en ce que** la concentration de suie pour des états de fonctionnement définis du moteur à combustion interne est acquise continuellement ou par intervalles sur la durée de fonctionnement, puis acheminée à un appareil d'interprétation (15) dans lequel est déterminé l'apport de suie dans l'huile moteur en fonction de la durée de fonctionnement et/ou en fonction de la charge.

6. Dispositif de détermination de la concentration de suie dans l'huile moteur de moteurs à combustion interne, notamment destiné à mettre en oeuvre un procédé selon l'une des revendications précédentes, comprenant :
- un segment de mesure servant à conduire une quantité définie de l'huile moteur à une vitesse d'écoulement définie ;
- au moins une source d'énergie (13) au moyen de laquelle de l'énergie peut être acheminée aux particules de suie dans l'huile moteur en au moins un point ou dans au moins une zone d'irradiation (14) défini(e) du segment de mesure ;
- un appareil de mesure de température destiné à acquérir une température (T1) en amont du point ou de la zone d'irradiation (14) ;
- un appareil de mesure de température destiné à acquérir plusieurs températures (T2, T3, T4) en aval du point ou de la zone d'irradiation (14) au moyen de plusieurs capteurs de température espacés les uns des autres dans l'espace, lesquels sont disposés dans un même plan de section transversal en référence au sens d'écoulement de l'huile moteur ; et
- un appareil d'interprétation (15) destiné à déterminer la concentration de suie en s'appuyant sur la distribution spatiale des plusieurs températures (T2, T3, T4) acquises.

7. Dispositif selon la revendication 6, **caractérisé en ce que** le segment de mesure est formé par une cuvette de circulation (2).

8. Dispositif selon la revendication 6, **caractérisé en ce que** la source d'énergie (13) est une source d'énergie optique, notamment une diode laser.
